# EUROPÄISCHE PATENTSCHRIFT

(11) **EP 2 964 294 B1**
(45) Veröffentlichungstag und Bekanntmachung des Hinweises auf die Patenterteilung: **11.12.2019**
(21) Anmeldenummer: 14712613.0
(22) Anmeldetag: 28.02.2014
(51) Int. Cl.: A61M 5/168, A61B 5/00, A61B 5/20, A61B 5/03, A61M 39/28

(54) **VORRICHTUNG ZUR REGULATION EINES VOLUMENSTROMS**
DEVICE FOR REGULATING A VOLUMETRIC FLOW RATE
DISPOSITIF DE RÉGULATION D'UN FLUX VOLUMIQUE

(30) Priorität: 04.03.2013 DE 102013102084
(43) Veröffentlichungstag der Anmeldung: 13.01.2016
(73) Patentinhaber: Medical Measurement Systems B.V., 7521 PV Enschede (NL)
(72) Erfinder: JENSEN, Michael Gondy, DK-4450 Jyderup (DK); LARSEN, Kristine, 81476 München (DE); VAN GORKOM, David, 83209 Prien am Chiemsee (DE); WITTE, Jens, 81476 München (DE)
(74) Vertreter: Wittmann, Ernst-Ulrich
(86) Internationale Anmeldenummer: PCT/EP2014/053970
(87) Internationale Veröffentlichungsnummer: WO 2014/135458

(56) Entgegenhaltungen:
- WO-A1-98/23320
- WO-A1-2012/161194
- US-A1- 2004 006 321

## Beschreibung

Die Vorliegende Erfindung betrifft eine Vorrichtung zur Regulation eines Volumenstroms, insbesondere für den Einsatz in der medizinischen Diagnose, insbesondere in der Urodynamik und der Gastroenterologie und einen Schlauchabschnitt zur Verwendung in einer entsprechenden Vorrichtung zur Regulation eines Volumenstroms.

Im Stand der Technik ist die diagnostische Druckmessung insbesondere in der Urodynamik bekannt. Bei der Druckmessung mit wassergefüllten Kathetern wird der Druck im Körper über eine Wassersäule, welche durch die Lumen der Katheter und die Druckübertragungsschläuche geführt wird, auf externe Drucksensoren geleitet. Dort misst man dann den Druck im Körper inkl. einen hydrostatischen Druck, der sich aus der Höhendifferenz der Messwertgeber zum Messort ergibt. Dieser Druckoffset wird entweder durch eine definierte Positionierung der Sensoren (Os Pubis-Höhe In der Urodynamik) oder durch elektronisches Nullsetzen kompensiert.

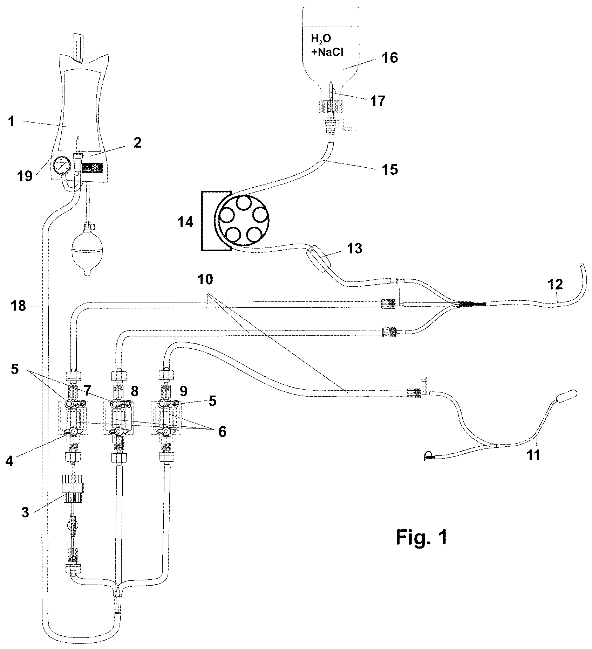

Heute bedarf eine urodynamische Untersuchung einen erheblichen Aufwand in der Vorbereitung und es wird eine erhebliche Anzahl an sterilen Einmalartikel benötigt. Ein Pumpenschlauch, ein Perfusionsschlauch, drei Druckmesswertgeber mit jeweils einem Drei- und einem Zweiwegehahn, drei Druckübertragungsschläuche, ein Transurethralkatheter mit einem Füll- sowie zwei Messvolumen und einem Rektalkatheter mit Ballon, welche alle am Messplatz aufgebaut und vorbereitet werden müssen (Figur 1).

Nachteilig ist hierbei, dass insbesondere auch eine Vielzahl von Hähnen vorgesehen werden müssen, die einerseits für die Vorbereitung der Untersuchung in unterschiedliche Positionen gestellt werden müssen, als auch für die Untersuchung nach einem vorgegebenen Ablaufplan geschaltet werden müssen.

Beispielweise, US 2004/006321 A1 beschreibt eine Vorrichtung zur Handhabung von Urin, insbesondere zur Durch- und Weiterleitung in einer Durchführung. Dabei ist in die Durchführung ein Abstandselement eingelegt, welches auch im Falle eines Knicks der Durchführung einen Mindestdurchfluss durch das Lumen der Durchführung gewährleistet.

WO 2012/161194 A1 beschreibt eine Infusionspumpe mit einem Schlauchabschnitt oder einer Durchführung, auf welche die beweglichen Rollen einer Rollenklemme in einer solchen Weise einwirken, dass mittels dieser Rollen die Durchführung blockiert und auch deren Durchfluss stufenlos von "blockiert" bis "offen" reguliert werden kann.

WO 98/23320 A1 beschreibt einen teilweise zusammenklappbaren Katheter, der sich als wesentlich abgeflachtes Rohr aus flexiblem zusammenklappbaren Kunststoff gebildet hat, wobei ein Abstandskatheter in einem solchen Rohr entsorgt wird.

Ausgehend von diesem Stand der Technik ist es nun Aufgabe der vorliegenden Erfindung eine Vorrichtung zur Regulation eines Volumenstroms bereit zu stellen, mit welchem die im Stand der Technik bekannten Nachteile wenigstens teilweise überwunden bzw. verbessert werden.

Gelöst wird diese Aufgabe durch ein erfindungsgemäßes Vorrichtung zur Regulation eines Volumenstroms gemäß Anspruch 1. Bevorzugte Ausgestaltungsformen der Vorrichtung und des Schlauchabschnittes sind Gegenstand der jeweiligen Unteransprüche. Mögliche Anwendungen der Erfindung: Regulation eines Volumenstroms und des entsprechenden Schlauchabschnittes im Bereich der Urodynamik und Gastroenterologie, insbesondere zur Blasen-, Rektal- und Urethraldruckmessung.

Die erfindungsgemäße Vorrichtung zur Regulation eines Volumenstroms umfasst wenigstens einen fluiddurchströmbaren Schlauchabschnitt und eine Schlauchklemme, wobei die Schlauchklemme ein Grundgehäuse mit einem Aufnahmebereich für den Schlauchabschnitt und ein, zwischen wenigstens zwei, bevorzugt zwischen drei Regulationsstellungen bewegbares Klemmelement aufweist. Das Klemmelement wirkt mit wenigstens einem Abschnitten des Aufnahmebereichs als Andruckstellen in wenigstens einer Regulationsstellung auf den Schlauchabschnitt und dessen Lumen ein, wobei im Bereich wenigstens einer Regulationsstellung im Lumen des Schlauchabschnitts ein Abstandshalter angeordnet ist.

Gemäß der vorliegenden Erfindung kann wenigstens die Vorrichtung zur Regulation des Volumenstroms in zwei bzw. drei Regulationsstellungen eingestellt werden, welche die Positionen "offen", "geschlossen" und/oder "perfundiert" umfasst. In der Position "offen" ist das Lumen des Schlauchabschnittes vollständig durchgängig, wo hingegen in der Position "geschlossen" das Lumen des Schlauchabschnittes vollständig geschlossen ist. In der perfundierten Stellung ist das Lumen des Schlauchabschnitts durch den Abstandshalten, der vorzugsweise im inneren des Lumens des Schlauchabschnitts angeordnet ist teilweise, insbesondere geringfügig offen.

Gemäß der vorliegenden Erfindung wird unter perfundiert d.h. geringfügig offen eine Einstellung verstanden, bei welcher unter vorgegebenen Bedingungen der Volumenstrom, welcher durch die Vorrichtung zur Regulierung des Fluidstroms bestimmbar ist, zwischen 0,1 ml/min und 8 ml/min vorzugsweise zwischen 0,5 ml/min und 5 ml/min begrenzt wird.

Solch eine Einstellung ist dann von Nöten, wenn insbesondere sehr geringe Volumenströme notwendig sind um die entsprechende Druckmessung in einem dynamischen System vornehmen zu können. Solche Volumenströme werden gemäß der vorliegenden Erfindung als geringfügig betrachtet, da sie im Vergleich zum normalen bzw. maximalen Volumenstrom als relativ klein zu bezeichnen sind.

Der Abstandshalter ist gemäß der vorliegenden Erfindung integraler Bestandteil des Schlauchabschnitts und/oder wird durch ein längliches, insbesondere ein faden- oder stabförmiges Element gebildet, welches wenigstens abschnittsweise mit dem Schlauchabschnitt kraft-, form- und/oder stoffschlüssig verbunden ist. Insbesondere dient der Abstandshalter zur Reduzierung des Lumens auf einen Wert zwischen 0,01 % bis 5 %, bevorzugt zwischen 0,1 % und 1 % und insbesondere bei ca. 0,5 %, so das hierdurch das Lumen des Schlauchabschnittes in der perfundierten Stellung geringfügig geöffnet und ein Volumenstrom zwischen 0,1 ml/min und 8 ml/min, vorzugsweise zwischen 0,5 ml/min und 5 ml/min bereitgestellt wird.

Der Abstandshalter wird ferner wenigstens teilweise aus einem Material hergestellt, das aus einer Gruppe ausgewählt ist, welche vorzugsweise Kunststoff, Wolle, Baumwolle, Cellulose, Metall, Kombinationen hiervon und dergleichen umfasst und weist insbesondere eine Querschnittsform auf, welche quadratisch, rechteckig, oval, kreisrund, vieleckig oder dergleichen ist. Darüber hinaus ist der Abstandshalter im Lumen des Schlauchabschnittes so angeordnet, dass das Lumen ungleichmäßig reduziert wird.

Gemäß einer weiteren, besonders bevorzugten Ausführungsform der vorliegenden Erfindung ist der Schlauchabschnitt aus einem flexiblen Material, insbesondere aus einem flexiblen Kunststoff gefertigt und abschnittsweise quetschbar ist, wobei ferner und vorzugsweise einen lichten Innendurchmesser aufweist, welcher zwischen 0,2 mm und 10 mm, vorzugsweise zwischen 0,5 mm und 5 mm und insbesondere bei ca. 3 mm liegt.

Gemäß einer weiteren, besonders bevorzugten Ausführungsform der vorliegenden Erfindung wird das Lumen des Schlauchabschnitts durch den Abstandshalter ungleichmäßig reduziert, d.h. des Lumen kann bei Quetschung nicht durch die Innenwände das Schlauchs vollständig geschlossen werden, sondern es bleibt wenigstens im unmittelbaren Bereich zum Abstandshalter ein Lumenbereich für das Fluid durchströmbar.

Als Fluid gemäß der vorliegenden Erfindung werden fließfähige Systeme insbesondere Flüssigkeiten betrachtet, die beispielsweise in der Medizin oder Medizintechnik verwendet werden. Solche sind beispielsweise Infusionslösungen wie Natriumchlorid Lösungen, Wasser, wässerige Lösungen, Injektionslösungen, Infusionslösungen, Nährlösungen, Elektrolytlösungen, Blut, Plasma, Gas, Luft, Kombinationen hieraus und dergleichen.

Diese Fluide werden gemäß einer weiteren besonders bevorzugten Ausführungsform in Fluidreservoiren bevorratet, die auch wiederrum im Stand der Technik wie beispielsweise in der Form von Infusionsflaschen oder Infusionsbeutel bekannt sind.

Gemäß einer besonders bevorzugten Ausführungsform der vorliegenden Erfindung ist die Vorrichtung zur Regulation eines Volumenstroms dadurch gekennzeichnet, dass die Regulationsstellungen durch wenigstens eine Schlauchklemme bewirkt werden, wobei das Klemmelement drehbeweglich angeordnet ist und sich radial um den Drehpunkt erstreckt. Insbesondere der zum Drehpunkt nächstgelegene Abschnitt des Klemmelements wirkt hierbei mit wenigstens einer Andruckstelle der Gehäuseaufnahme beziehungsweise des Gehäuseaufnahmebereiches als Gegendruckelement zusammen.

In einer alternativen, aber auch besonders bevorzugten Ausführungsform wird das Klemmelement mittels eines Motors, insbesondere eines Schrittmotors oder eines Servomotors zwischen den Regulationsstellungen bewegt und vorzugsweise in diesen entsprechend gehalten bzw. arretiert. Hierbei können für die einzelnen Positionen Arretiermechanismen vorgesehen sein oder der entsprechende Schrittmotor oder Servomotor bietet an den entsprechenden Positionen Arretierungen. Alternativ hierzu kann das Klemmelement auch händisch in entsprechende Positionen bewegt werden und ggf. mechanisch fixiert werden.

In einer weiteren, besonders bevorzugten Ausführungsform der vorliegenden Erfindung wird die Vorrichtung zur Regulation eines Volumenstroms, die Schlauchklemme bzw. der Schlauchabschnitt oder wenigstens Teile hiervon aus einem Material hergestellt, das aus einer Gruppe ausgewählt ist, welche vorzugsweise duro- und thermoplastische Kunststoff und insbesondere Polyphenylensulfid, Polypropylen, Poly-1-buten, Polyvinylchlorid, Polyvinylidenchlorid, Polymethyl-metaacrylat, Polyacrylnitril, Polystyrol, Polyacetal, Polyvinylalkohol, Polyvinylacetat, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurthan, ungesättigtes Polyesterharz, Acrylnitril-Butadien-Styrol, Acrylester-Styrol-Acrylnitril, Metalle wie zum Beispiel Edelstahl, Aluminium, Kombinationen hiervon und dergleichen umfasst.

Nachfolgend wird die Erfindung anhand eines bevorzugten Ausführungsbeispiels beschrieben, wobei darauf hingewiesen wird, dass die Erfindung nicht auf die hier dargestellte Ausführung beschränkt, sondern vielmehr auch entsprechende Abwandlungen im Sinne der vorliegenden Erfindung sind.

Dabei zeigen:
- Figur 1: den Aufbau eines Messsystem gemäß dem Stand der Technik;
- Figur 2: den Aufbau eines Druckmesssystems mit einer erfindungsgemäßen Vorrichtung zur Regulation eines Volumenstroms;
- Figuren 3a - 3h: Detailansichten der Vorrichtung zur Regulation des Volumenstroms;
- Figur 4: eine Detailansicht des Pumpenschlauchs für das erfindungsgemäße Druckmesssystems;
- Figur 5: Messprotokoll einer automatischen Entlüftung und Nullstellung des erfindungsgemäßen Druckmesssystems.

In Figur 1 ist schematisch der Aufbau einer Vorrichtung zur Druckmessung mit wassergefüllten Einmalkathetern dargestellt. Dabei ist neben dem Beutel mit einer Infusionslösung 1 und einer Druckmanschette 2, ein Druckminderer 3 und Zweiwegehähne 4 vor den Drucksensoren 6 dargestellt. Im Anschluss hieran sind Dreiwegehähne 5 angeordnet und die entsprechenden Leitungen werden als Pᵤᵣₐ 7, Pᵥₑₛ 8 und P_{abd} 9 bezeichnet. Über die Druckübertragungsschläuche 10 wird die Infusionslösung aus dem Beutel 1 zu dem Rektalkatheter 11 und den UDP-Katheter 12 geleitet. Darüberhinaus wird zum UDP-Katheter 12 aus der Flasche 16 mit z.B. einer Kochsalzlösung über die Rollenpumpe 14 und die Tropfkammer 13 die Kochsalzlösung aus der Flasche 16 definiert zugeführt.

Hiermit wird für die Vorbereitungs- und Messprozedur wie folgt verfahren:
a) Der Pumpenschlauch 15 wird in die Pumpe 14 eingelegt und der Dorn 12 des Schlauchs in den Flaschenstopfen eingesetzt. Die Pumpe 14 wird eingeschaltet bis die Tropfenkammer 13 der Flasche 16 halb gefüllt und der Schlauch komplett luftblasenfrei mit Kochsalzlösung gefüllt ist.
b) Die drei Druckmesswertgeber 6 werden in den Halterungen platziert und mit dem Perfusionsschlauch 18 verbunden - zwischen den Perfusionsschlauch und einen Druckmesswertgeber wird ein Durchflußminderer 3 eingesetzt. Der Dorn 19 des Perfusionsschlauchs 18 wird in die Wasserbeutel 1 eingesteckt.
c) Die Druckübertragungsschläuche 10 werden mit den Druckmesswertgebern 6 verbunden.
d) Alle Zwei- 4 und Dreiwegehähne 5 werden geschlossen und die Druckmanschette 2 wird aufgepumpt um die Wasserbeutel 1 unter Druck zu setzen.
e) Zum Entlüften der Druckübertragungslinien werden die Zwei- 4 und Dreiwegehähne 5 der Drucktransducer auf "offen" gestellt und blasenfrei bis an die Spitze mit Wasser gefüllt und anschließend der Zweiwegehahn 2 wieder geschlossen. Diese Prozedur muss für alle drei Druckübertragungslinen einzeln durchgeführt werden.
f) Die Dreiwegehähne 5 werden jetzt einzeln in "90 grad"- Position gedreht um die Druckkanäle per Tastendruck elektronisch auf Atmosphäre abzugleichen. Die Dreiwegehähne 5 werden anschließend wieder auf die Position "offen" gedreht.
g) Die beiden Katheter 11, 12 werden in Urethra und Rektum des Patienten platziert und an die Druckübertragungslinien sowie den Pumpenschlauch 10 angeschlossen.
h) Die Dreiwegehähne 5 müssen jetzt noch mal einzeln geöffnet um die beiden Lumen des Transurethralkathers 12 bis an die Spitze zu entlüften und den Ballon des Rektalkatheters 11 zu füllen.
i) Die gemessene Drücke werden überprüft und wenn notwendig per Software auf Null gestellt. Das System ist jetzt messbereit.

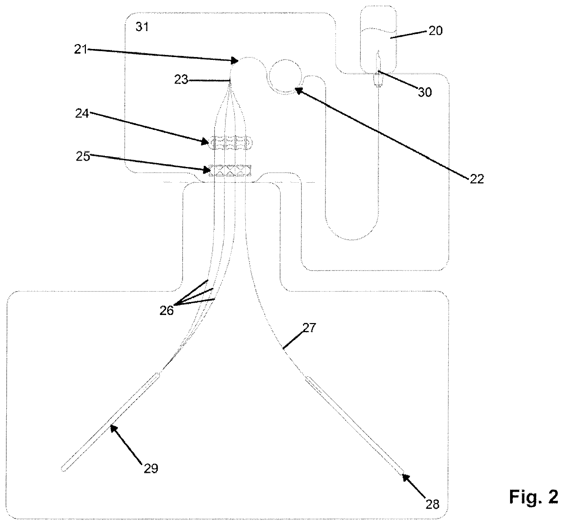

In Figur 2 ist der Aufbau eines erfindungsgemäßen Druckmesssystems dargestellt.

Dieses zeigt mit dem Bezugszeichen 21 den Pumpenschlauch des zu der Druckdomkassette 25 mit der Luer-Locks für den Anschluss von Kathetern 28 und 29 führt. Ferner ist der Einstichdorn 30 für Standard-Infusionbeutel 20 dargestellt. Mit dem Bezugszeichen 29 ist der Transurethraler und mit dem Bezugszeichen 28 der Rektalkatheter (ggf. auch beliebig viele Katheter oder Messvolumen), bezeichnet, welche mit dem Fluid über die Leitungen 26 (drei Stufen) und 27 versorgt werden. Hieran anschließend ist der Verriegelungsmechanismus der Domkassette 25 angeordnet. Im Bereich des Bezugszeichens 24 sind vorliegend vier (ggf. beliebig viele) Schlauchklemmen mit den Zuständen "offen", "geschlossen" und "perfundiert" angeordnet. Mit dem Bezugszeichen 22 ist eine Rollpumpe zur Beförderung des Mediums durch das Schlauchsystem dargestellt. Das System umfasst ferner ein Steuerungssystem 31 mit vollautomatischen Algorithmus zur Entlüftung des Schlauchsystems mit Kathetern, Auffindung von Ruhedrücken und Nullstellung des Messsystems unter Ausnutzung der Funktionalität der Einzelkomponenten.

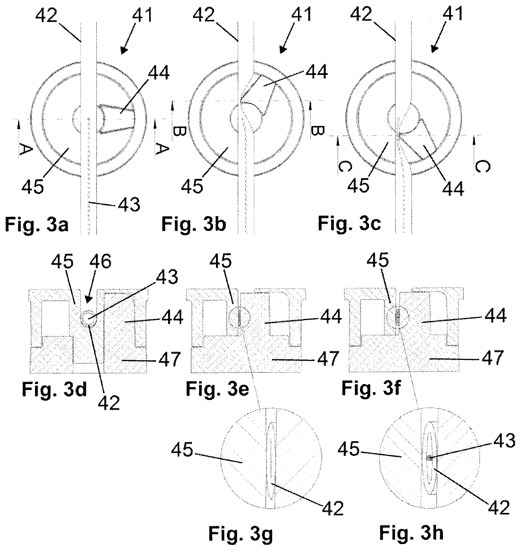

In den Figuren 3a - 3h sind unterschiedliche Stellungen (Offen, Geschlossen, Perfundiert) der Vorrichtung 41 zur Regulierung des Volumenstroms d.h. der Schlauchklemme gezeigt.

Hierbei ist in der oberen Reihe 3a bis 3c die Draufsicht auf eine entsprechende Vorrichtung 41 gezeigt, bei welcher der Schlauch 42 eingelegt ist. Im Schlauch ist in der unteren Hälfte ein Faden 43 im Sinne eines Abstandshalters zu erkennen. Ferner ist das Klemmelement 44 und die hiermit zusammenwirkbare Gegenwand 45 der Vorrichtung 41 dargestellt. In Figur 3a ist die offene Stellung, in der Figur 3b die vollständig geschlossene Stellung und in der Figur 3c die sogenannte perfundierte, d.h. die geringfügig geöffnete Stellung wiedergegeben.

In den Figuren 3d bis 3f sind Schnittdarstellungen aus den Figuren 3a bis 3c entlang der jeweiligen Schnittlinien A-A, B-B und C-C wiedergegeben. Auch hier ist der eingelegte Schlauch 42 mit dem Abstandshalter 43 zu erkennen, der in der Aufnahme 46 der Vorrichtung 41 eingelegt ist. Mit dem Bezugszeichen 44 ist das Klemmelement bezeichnet, welches auf einer drehbar gelagerten Führung 47 angeordnet ist. Mit dem Wandabschnitt 45 kann dieses in den entsprechenden Positionen (siehe 3c und 3f) zusammenwirken, wobei entsprechend der hier gezeigten Darstellung in der Position nach Figur 3b und 3e der Schlauch vollständig geschlossen ist und in der Position nach den Figuren 3c und 3f der Schlauch geringfügig geöffnet ist, obwohl das Klemmelement 44 mit der Gegenwand 45 zusammenwirkt. Das Abstandselement 43 bewirkt aber einen geringfügigen Fluidstrom trotz der bestehenden Quetschung des Schlauchs. In den Figuren 3g und 3h sind Detaildarstellungen der beiden Positionen 3e und 3h wiedergegeben, um den Unterschied zwischen geschlossen und perfundiert zu veranschaulichen. Deutlich ist die geringfügige Öffnung des Schlauches in der Fig. 3h zu erkennen.

Solch eine Vorrichtung 41 kann auch als Schlauchklemme bezeichnet werden. Die Schlauchklemmen ersetzen die in der konventionellen Druckmesstechnik verwendeten Zwei- und Dreiwegehähne. Zusätzlich erfüllen sie auch die Funktion des Perfusionsreglers für Druckmesskanäle, die perfundiert werden müssen (Urethradruck in der Urodynamik). Die Klemmen können durch einfaches Drehen der Achse mit einem beliebigen Aktuator in die Positionen "offen", "geschlossen" und "perfundiert" gebracht werden.

Position "offen": In dieser Position kann das Füllmedium die Schlauchklemmen ungehindert passieren. Sie ist die Start- und Endposition für alle Kanäle in der sich das Schlauchsystem einfach einlegen bzw. entnehmen lässt, wird zusätzlich für die Entlüftung der Messkanäle verwendet und stellt den normalen Arbeitszustand für den Infusionskanal dar.

Position "geschlossen": In dieser Position wird der Schlauch vollständig abgeklemmt und ist auch bei Druckdifferenzen von bis zu 2 bar unpassierbar für das Fluid. Sie ist der Arbeitszustand für die bereits entlüfteten Messkanäle und der vorübergehende Initialzustand für den Infusionskanal, wenn die Messkanäle entlüftet oder perfundiert werden.

Position "perfundiert": In dieser Position können, weitgehend unabhängig vom Vordruck, nur sehr geringe Mengen des Füllmediums (max. 8 ml/min) die Klemmstelle passieren. Das wird dadurch erreicht, indem der Schlauch zwar ähnlich wie in der Position "geschlossen" mit voller Kraft abgeklemmt wird, jedoch die Wände der Schläuche durch einen Abstandshalter wie zum Beispiel einen Faden immer eine kleine Öffnung freigehalten wird, die eine kapillaren Reduzierung des Füllmedienstroms bewirkt. Diese Position wird für Kanäle verwendet, die Perfusion erfordern (Urethadruck).

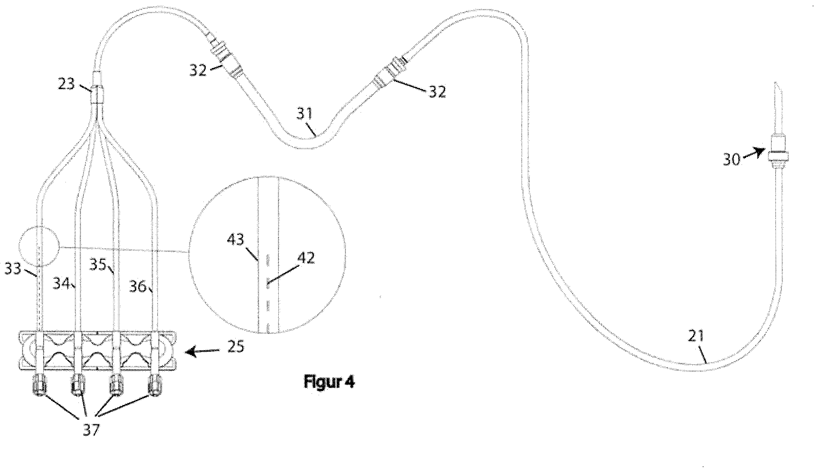

Im Pumpenschlauch - wie in Figur 4 gezeigt - sind der Infusionsschlauch 21, die Entlüftungsschläuche 34 bis 36 für die drei - ggf. beliebig viele - Druckkanäle und die Domkassette 25 integriert. Zudem besitzt der Pumpen- bzw. Infusionsschlauch 21 einen Anstichdorn 30 für einen Infusionsbeutel und Luer-Locks 37 für den Anschluss der Katheter. Bei der Verwendung des Systems mit einer Rollenpumpe weist das Schlauchsystem in diesem Bereich einen geeigneten kompressiblem Schlauchabschnitt 31 auf, der durch entsprechende Verbinder 32 in das Schlauchsystem integriert ist. Über den Verteiler 23 wird das Schlauchsystem auf die hier dargestellten vier Schläuche 33 bis 36 aufgeteilt. Der Schlauch 33 umfasst neben dem eigentlichen Schlauch 42 auch den Abstandshalter 43, wie dies in der Detaildarstellung der Fig. 4 zu erkennen ist.

Besonders vorteilhaft ist bei der vorliegenden Erfindung die Integration von drei - ggf. beliebig vielen - Domen in eine Domkassette und die gemeinsame Kopplung der Kanäle an die Sensoren über eine Andruckwalze und Klemmkante. Hierbei wirken die auf Andruckwalzen- und Klemmkantenseite in die Domkassette integrierten Stege als Federelemente, die den benötigten Anpressdruck der Membrane, die vorzugsweise auf den Bodenabschnitt der Druckdome angeordnet sind, auf die Sensoren herstellen.

Diese Konstruktion hat große Vorteile gegenüber der konventionellen Lösung mit Einzeldomen: Die Herstellung ist sehr viel günstiger und die Handhabung wird extrem vereinfacht. Statt wie bei den bisherigen Lösungen jeden Dom einzeln auf seinen Sensor zu stecken, genügt das Einlegen der Kassette und ein anschließender Knopfdruck mit dem die Anpresswalze über einen Aktuator in die "geschlossen" Position gedreht wird. Die vormals hohe Anzahl von benötigten, sterilen Einzelkomponenten ist insbesondere auf ein einziges Einmalprodukt reduziert.

Da in vielen Anwendungsgebieten die Sensoren oberhalb der Messorte im Körper platziert sein können, entstehen wegen der hydrostatischen Kraft der Wassersäule auch negative Drücke (niedriger als Atmosphärendruck) im Dom, die dann nicht durch Druck auf die Sensorfläche, sondern durch Zug gemessen werden. Zur Herstellung des dazu benötigten Saugeffektes müssen die Kontaktierungen zwischen Membranen und Sensoren komplett luftdicht ausgelegt sein. Dazu benötigt man einen Anpressdruck.

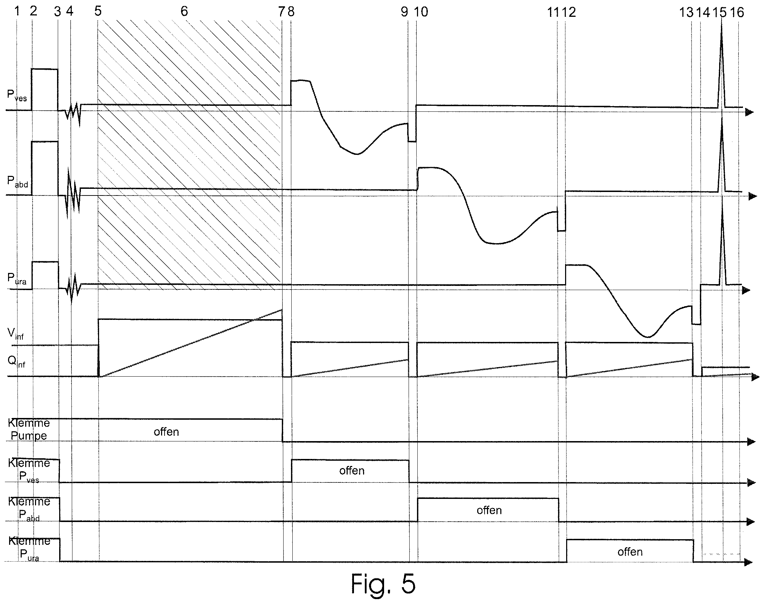

In Figur 5 ist über die Verfahrensschritte der Druckverlauf an den entsprechenden Positionen bzw. die Stellposition der Bauteile wiedergegeben. Dabei steht Pᵤᵣₐ, Pᵥₑₛ und P_{abd} für den Druck in den entsprechenden Druckdomen, V_{inf} und Qi_{nf} für das Infusionsvolumen und den Fluidstrom und Klemme-Pumpe, Klemme-Pᵤᵣₐ, Klemme-P_{abd} und Klemme-Pᵥₑₛ für die Schaltposition der entsprechenden Schlauchklemme. Das Verfahren wird nachfolgend beispielhaft beschrieben:
1) Der Benutzer legt die Domkassette 25 des Pumpenschlauchs 21 in das Gerät ein und führt den Einstichdorn 30 in den Infusionsbeutel 20 ein.
2) Der Verriegelungsmechanismus wird geschlossen, wodurch durch den Anpressdruck der Dome 25 auf die Sensoren ein Druckoffset entsteht.
3) Der Druckoffset wird automatisch nach einer Materialentspannungszeit von 5-10s ausgeglichen (zu Null gesetzt). Gleichzeitig werden die Schlauchklemmen 24 der Druckkanäle 26 und 27 geschlossen.
4) Der Benutzer platziert den transurethralen Katheter 29 in die Blase und den rektalen Katheter 28 in das Rektum des Patienten (falls nicht schon vorher geschehen) und verbindet den Pumpenschlauch 21 sowie die Druckübertragungsschläuche mit den Luer-Locks der Domkassette 25. Ab diesem Zeitpunkt kann der Benutzer die korrekte Platzierung der Katheter 28,29 anhand der am Gerät angezeigten Ruhedruckwerte, die jetzt über die Luftsäule der Druckübertragungsschläuche und Katheterlumen gemessen werden, überprüfen.
5) Wenn die Ruhedrücke plausible Werte zeigen, startet der Benutzer die vollautomatische Entlüftungs- und Nullstellungsprozedur per Tastendruck am Gerät, bei der zunächst die gesamte Infusionslinie entlüftet wird.
6) Im schraffiert dargestellten Zeitintervall messen alle Druckkanäle den Ruhedruck am jeweiligen Messort über die Luftsäule. Um zufällige Schwankungen durch Bewegung des Patienten oder Störungen zu minimieren wird der durchschnittlich gemessene Wert aller Kanäle während dieses Zeitraums berechnet und später als Ruhedruckwert zur Nullstellung verwendet.
7) Da das Volumen der Infusionslinie (Schlauch plus Fülllumen des Katheters) exakt bekannt ist und die beförderte Menge des Füllmediums durch den Volumentransducer bzw. Umdrehungen des Rollenrades der Pumpe 22 ständig gemessen wird, kann die Entlüftung vollautomatisch beendet werden, wenn die Wassersäule den Fülllumenausgang erreicht hat. Die Pumpenschlauchklemme 24 wird geschlossen.
8, 10, 12) Die Entlüftung der Druckübertragungsschläuche geschieht dann sequentiell immer nach dem gleichen Schema. Die jeweilige Schlauchklemme wird geöffnet, während alle anderen geschlossen sind und die Pumpe befördert das Füllmedium in definierter Füllgeschwindigkeit durch die jeweilige Druckübertragungslinie. Zu beachten ist hierbei, dass während des Befüllens wegen der sich aufbauenden Wassersäule zunehmend eine hydrostatische und zusätzlich eine dynamische Komponente hinzukommt, nicht mehr der tatsächliche Druck am Messort ermittelt wird..
9, 11, 13) Da die Volumina der Druckübertragungslinien bekannt sind und die Füllmenge ständig gemessen wird, kann der Entlüftungsvorgang vollautomatisch beendet werden, wenn die Wassersäule die Messlumenausgänge erreicht hat. Die Pumpe 22 wird gestoppt und die jeweilige Schlauchklemme 24 geschlossen. In diesem Moment misst das Gerät den momentanen Druck am Messort im Körper plus einen sich aus dem nicht bekannten Höhenunterschied zwischen Messort und Sensor ergebenden hydrostatischen Druck. Dieser wird jetzt vollautomatisch herausgerechnet, indem man softwaremäßig den jeweiligen Messkanal auf den in (6) über Luftsäule gemessenen Ruhedruck setzt.
14, 15) Die korrekte Platzierung der Katheter wird in einem letzten Schritt überprüft, indem man den Patienten Husten lässt und die dadurch entstehenden Druckspitzen vergleicht. Dazu werden die Schlauchklemmen 24 aller Kanäle, die Perfusion für die Messung benötigen (Pura in der Urodynamik), automatisch in die Perfusionstellung und die Pumpe 22 auf Perfusionsgeschwindigkeit gestellt.
16) Die Vorbereitungsphase ist abgeschlossen und die eigentliche Messung kann beginnen.

## Patentansprüche

1. Vorrichtung (41) zur Regulation eines Volumenstroms mit einem fluiddurchströmbaren Schlauchabschnitt (42) und einer Schlauchklemme, wobei die Schlauchklemme ein Grundgehäuse mit einem Aufnahmebereich für den Schlauchabschnitt (42) und ein, zwischen wenigstens zwei, bevorzugt in drei Regulationsstellungen bewegbares Klemmelement (44) aufweist,
**dadurch gekennzeichnet, dass**
das Klemmelement (44) in Wechselwirkung mit Abschnitten des Aufnahmebereichs als Andruckstellen in wenigstens einer Regulationsstellung auf den Schlauchabschnitt (42) und dessen Lumen einwirkt,
das Klemmelement (44) drehbeweglich angeordnet ist und sich das Klemmelement (44) radial um den Drehpunkt erstreckt und insbesondere der zum Drehpunkt nächstgelegene Abschnitt des Klemmelements (44) mit wenigstens einer Andruckstelle zusammenwirkt,
im Bereich wenigstens einer Regulationsstellung im Lumen des Schlauchabschnitts (42) ein Abstandshalter (43) angeordnet ist, und
die Vorrichtung (41) wenigstens in die Regulationsstellungen offen, geschlossen und perfundiert einstellbar ist, wobei in der geschlossenen Stellung das Lumen des Schlauchabschnitts (42) vollständig geschlossen und in der perfundierenden Stellung das Lumen des Schlauchabschnitts (42) durch den Abstandhalter (43) im Inneren des Schlauchabschnitts teilweise, insbesondere geringfügig offen ist.

2. Vorrichtung (41) gemäß Anspruch 1, **dadurch gekennzeichnet, dass**
die Andruckstellen durch wenigstens ein Gegendruckelement für das Klemmelement (44) gebildet werden.

3. Vorrichtung (41) gemäß einem der vorstehenden Ansprüche, **dadurch gekennzeichnet, dass**
das Klemmelement (44) mittels eines Motors, insbesondere einem Schrittmotor oder Servomotor, zwischen den Regulationsstellungen bewegt und in diesen gehalten wird.

4. Vorrichtung (41) gemäß einem der vorstehenden Ansprüche, **dadurch gekennzeichnet, dass**
die Vorrichtung, die Schlauchklemme bzw. der Schlauchabschnitt (42) oder Teile hiervon wenigstens abschnittsweise aus einem Material hergestellt wird, das aus einer Gruppe ausgewählt ist, welche vorzugsweise duro- und thermoplastische Kunststoffe und insbesondere Polyphenylensulfid, Polypropylen, Poly-1-buten, Polyvinylchlorid, Polyvinylidenchlorid, Polymethyl-metaacrylat, Polyacrylnitril, Polystyrol, Polyacetal, Polyvinylalkohol, Polyvinylacetat, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurthan, ungesättigtes Polyesterharz, Acrylnitril-Butadien-Styrol, Acrylester-Styrol-Acrylnitril, Metalle wie zum Beispiel Edelstahl, Aluminium, Kombinationen hiervon und dergleichen umfasst.

5. Vorrichtung (41) gemäß einem der vorstehenden Ansprüche, **dadurch gekennzeichnet, dass**
das Fluid aus einer Gruppe ausgewählt wird, welche Wasser, wässrige Lösungen, Injektionslösungen, Infusionslösungen, Nährlösungen, Elektrolytlösung, Blut, Plasma, Gase, Luft, Kombinationen hieraus und dergleichen aufweist.

6. Vorrichtung (41) gemäß einem der vorstehenden Ansprüche, **dadurch gekennzeichnet, dass**
der Schlauchabschnitt (42) aus einem flexiblen Material, insbesondere aus einem flexiblen Kunststoff, gefertigt ist und abschnittsweise quetschbar ist.

7. Vorrichtung (41) gemäß einem der vorstehenden Ansprüche, **dadurch gekennzeichnet, dass**
der Schlauchabschnitt (42) ein Lumen d.h. einen lichten Innendurchmesser aufweist, welcher zwischen 0,2 mm und 10 mm, vorzugsweise zwischen 0,5 und 5 mm und insbesondere bei ca. 3 mm, liegt.

8. Vorrichtung (41) gemäß einem der vorstehenden Ansprüche, **dadurch gekennzeichnet, dass**
der Abstandhalter (43) integraler Bestandteil eines Schlauchabschnitts ist und insbesondere in diesem Abschnitt das Lumen des Schlauchs (42) reduziert und/oder der Abstandhalter durch ein längliches, insbesondere ein faden- oder stabförmiges, das Lumen des Schlauchs (42) reduzierendes Element gebildet, welches wenigstens abschnittsweise mit dem Schlauchabschnitt (42) kraft-, form- und/oder stoffschlüssig verbunden ist.

9. Vorrichtung (41) gemäß einem der Ansprüche 1 bis 8, **dadurch gekennzeichnet, dass**
die Reduzierung des Lumens durch den Abstandhalter (43) zwischen 0,01 % bis 5 %, bevorzugt zwischen 0,1 % und 1 % und insbesondere bei ca. 0,5 %, liegt und dadurch das Lumen des Schlauchabschnittes in der perfundierten Stellung derart geringfügig offen gehalten wird, dass ein Volumenstrom des Fluids zwischen 0,1 ml/min und 8 ml/min, vorzugsweise zwischen 0,5 ml/min und 5 ml/min, bereitgestellt wird.

## Claims

1. Device (41) for regulating a volume flow, comprising a tube portion (42) through which liquid can flow, and a tube clamp, the tube clamp comprising a main housing having a receiving region for the tube portion (42) and a clamping element (44) that can be moved between at least two, preferably into three regulating positions, **characterised in that** the clamping element (44), in interaction with portions of the receiving region as pressure points, acts on the tube portion (42) and its lumen in at least one regulating position, the clamping element (44) is rotatably mounted and the clamping element (44) extends radially around the point of rotation and in particular the portion of the clamping element (44) nearest to the point of rotation interacts with at least one pressure point, a spacer (43), in the region of at least one regulating position, is arranged in the lumen of the tube portion (42), and the device (41) can be set at least into the three regulating positions, namely open, closed and perfusing, the lumen of the tube portion (42) being completely closed in the closed position, and, in the perfusing position, the lumen of the tube portion (42) being partly, in particular slightly open by means of the spacer (43) inside the tube portion.

2. Device (41) according to claim 1, **characterised in that** the pressure points are formed by at least one counterpressure element for the clamping element (44).

3. Device (41) according to either of the preceding claims, **characterised in that** the clamping element (44) is moved by means of a motor, in particular a stepper motor or servomotor, between the regulating positions and is held therein.

4. Device (41) according to any one of the preceding claims, **characterised in that** the device, the tube clamp and the tube portion (42) or parts thereof are made, in portions, from at least one material, selected from a group that comprises preferably duroplast and thermoplastic materials and in particular polyphenylene sulfide, polypropylene, poly-1-butene, polyvinyl chloride, polyvinylidene chloride, polymethyl methacrylate, polyacrylonitrile, polystyrene, polyacetal, polyvinyl alcohol, polyvinyl acetate, ionomers, fluoroplastic, polyethylene, polyamide, in particular a partly aromatic polyamide, polycarbonate, polyester, polyphenylene oxide, polysulfone, polyvinyl acetal, polyurethane, and chlorinated polyether, cellulose nitrate, cellulose acetate, cellulose ether, phenol resin, urea resin, thiourea resin, melamine resin, alkyl resin, allyl resin, silicone, polyimide, polybenzimidazole, epoxy resin, casein plastic, cross-linked polyurethane, unsaturated polyester resin, acrylonitrile butadiene styrene, acrylic ester styrene acrylonitrile, metals such as stainless steel, aluminium, combinations thereof and the like.

5. Device (41) according to any one of the preceding claims, **characterised in that** the fluid is selected from a group comprising water, aqueous solutions, injection solutions, infusion solutions, nutrient solutions, electrolyte solution, blood, plasma, gases, air, combinations thereof and the like.

6. Device (41) according to any one of the preceding claims, **characterised in that** the tube portion (42) is made from a flexible material, in particular from a flexible plastics material, and can be squeezed in portions.

7. Device (41) according to any one of the preceding claims, **characterised in that** the tube portion (42) has a lumen, i.e. a clear inner diameter that is between 0.2 mm and 10 mm, preferably between 0.5 and 5 mm and in particular is approximately 3 mm.

8. Device (41) according to any one of the preceding claims, **characterised in that** the spacer (43) is an integral part of a tube portion and, in particular in this portion, it reduces the lumen of the hose (42), and/or the spacer is formed by an elongate, in particular a thread-like or rod-like element that reduces the lumen of the tube (42), which element is, at least in portions, force-lockingly connected, form-fittingly connected and/or integrally bonded to the tube portion (42).

9. Device (41) according to any one of claims 1 to 8, **characterised in that** the reduction of the lumen by the spacer (43) is between 0.01% and 5%, preferably between 0.1% and 1% and in particular is approximately 0.5%, and, as a result, the lumen of the tube portion in the perfusing position is held slightly open in such a way that a volume flow of the fluid between 0.1 ml/min and 8 ml/min, preferably between 0.5 ml/min and 5 ml/min, is provided.

## Revendications

1. Dispositif (41) de régulation d'un flux volumique comprenant un segment de tuyau pouvant être traversé par un fluide (42) et un collier de serrage, le collier de serrage possédant un boîtier de base avec une zone de réception pour le segment de tuyau (42) et un élément de serrage (44) mobile entre deux, ou de préférence trois positions de régulation,
**caractérisé en ce que**
l'élément de serrage (44) agit sur le segment de tuyau (42) et sa lumière en interaction avec des sections de la zone de réception faisant office de points de pression dans au moins une des positions de régulation,
l'élément de serrage (44) est disposé de façon rotative et l'élément de serrage (44) s'étend radialement autour du point de rotation et en particulier la section de l'élément de serrage (44) la plus proche du point de rotation interagit avec au moins un point de pression,
dans la région d'au moins une position de régulation, un écarteur (43) est disposé dans la lumière du segment de tuyau (42) et
le dispositif (41) peut être mis en position ouverte, fermée et de perfusion au moins dans les positions de régulation, la lumière du segment de tuyau (42) étant complètement fermée dans la position fermée et la lumière du segment de tuyau (42) étant partiellement, en particulier légèrement ouverte, par l'écarteur (42) disposé à l'intérieur en position de perfusion.

2. Dispositif (41) selon la revendication 1, **caractérisé en ce que**
les points de pression sont formés par au moins un élément de contre-pression pour l'élément de serrage (44).

3. Dispositif (41) selon une des revendications précédentes, **caractérisé en ce que**
l'élément de serrage (44) est mu entre les positions de régulation et maintenu dans celles-ci au moyen d'un moteur, en particulier d'un moteur pas-à-pas ou d'un servomoteur.

4. Dispositif (41) selon une des revendications précédentes, **caractérisé en ce que**
le dispositif, le collier de serrage et le segment de tuyau (42) ou des parties de ceux-ci sont fabriqués au moins partiellement dans un matériau qui est sélectionné dans un groupe qui comprend de préférence des matières thermodurcissables et thermoplastiques et en particulier du polysulfure de phénylène, du polypropylène, du poly-1-butène, du polychlorure de vinyle, du polychlorure de vinylidène, du polyméthacrylate de méthyle, du polyacrylonitrile, du polystyrène, du polyacétal, de l'alcool polyvinylique, de l'acétate de polyvinyle, des ionomères, du plastique fluoré, du polyéthylène, du polyamide, en particulier un polyamide partiellement aromatique, du polycarbonate, du polyester, de l'oxyde de polyphénylène, de la polysulfone, de l'acétal de polyvinyle, du polyuréthane et du polyéther chloré, du nitrate de cellulose, de l'acétate de cellulose, de l'éther cellulosique, de la résine phénolique, de la résine d'urée, de la résine de thiourée, de la résine de mélamine, de la résine alkyde, de la résine allilique, du silicone, du polyimide, du polybenzimidazole, de la résine époxy, du plastique de caséine, du polyuréthane réticulé, de la résine polyester insaturée, de l'acrylonitrile-butadiène-styrène, de l'acrylonitrile-ester-acrylate, des métaux tels que, par exemple, de l'acier inoxydable, de l'aluminium, des combinaisons de ceux-ci et autres.

5. Dispositif (41) selon une des revendications précédentes, **caractérisé en ce que**
le fluide est sélectionné dans un groupe qui comprend de l'eau, des solutions aqueuses, des solutions injectables, des solutions perfusables, des solutions nutritive, une solution électrolytique, du sang, du plasma, des gaz, de l'air, des combinaisons de ceux-ci et autres.

6. Dispositif (41) selon une des revendications précédentes, **caractérisé en ce que**
le segment de tuyau (42) est fabriqué dans une matière flexible, en particulier dans une matière plastique flexible, et est partiellement compressible.

7. Dispositif (41) selon une des revendications précédentes, **caractérisé en ce que**
le segment de tuyau (42) possède une lumière, c'est-à-dire un diamètre intérieur qui mesure entre 0,2 mm et 10 mm, de préférence entre 0,5 mm et 5 mm et en particulier environ 3 mm.

8. Dispositif (41) selon une des revendications précédentes, **caractérisé en ce que**
l'écarteur (43) fait partie intégrante d'un segment de tuyau et réduit la lumière du tuyau (42) en particulier dans ce segment et/ou l'écarteur est formé par un élément oblong, en particulier en forme de fil ou de tige, réduisant la lumière du tuyau (42), qui est relié au moins partiellement par friction, mécaniquement et/ou par combinaison de matière avec le segment de tuyau (42).

9. Dispositif (41) selon une des revendications 1 à 8, **caractérisé en ce que**
la réduction de la lumière par l'écarteur (43) est de 0,01% à 5%, de préférence de 0,1% à 1% et en particulier d'environ 0,5%, et que, de ce fait, la lumière du segment de tuyau en position de perfusion est maintenue légèrement ouverte de sorte à obtenir un débit du fluide de 0,1 ml/min à 8 ml/min, et en particulier de 0,5 ml/min à 5 ml/min.
